Europäisches Patentamt

European Patent Office

Office européen des brevets

(11) Publication number: **0 499 162 A2**

(12) **EUROPEAN PATENT APPLICATION**

(21) Application number: **92102105.1**

(22) Date of filing: **07.02.92**

(51) Int. Cl.5: **C07K 7/08, A61K 37/02**

(30) Priority: **11.02.91 US 653427**

(43) Date of publication of application:
**19.08.92 Bulletin 92/34**

(84) Designated Contracting States:
**AT BE CH DE DK ES FR GB GR IT LI LU MC NL PT SE**

(71) Applicant: **Bristol-Myers Squibb Company**
**345 Park Avenue**
**New York, N.Y. 10154(US)**

(72) Inventor: **Bursuker, Isia**
**29 Currier Way**
**Cheshire, CT 06410(US)**
Inventor: **Greenfield, Robert S.**
**10 Seiter Hill Road**
**Wallingford, CT 06492(US)**
Inventor: **Braslawsky, Gary R.**
**124 Heritage Drive**
**Glastonbury, CT 06033(US)**

(74) Representative: **Kinzebach, Werner, Dr. et al**
**Patentanwälte Reitstötter, Kinzebach und**
**Partner Sternwartstrasse 4 Postfach 86 06 49**
**W-8000 München 86(DE)**

(54) **GM-CSF inhibiting oligopeptides.**

㊐ Provided are oligopeptides which are capable of inhibiting the biological activity of a granulocyte-macrophage colony stimulating factor ("GM-CSF"). These oligopeptides are useful for alleviating undesirable biological effects mediated by GM-CSF. In particular, the oligopeptides are useful for inhibiting the growth of a GM-CSF dependent neoplastic disease. Also provided are formulations and methods for using the oligopeptides of the invention.

EP 0 499 162 A2

Colony stimulating factors ("CSFs") are a group of polypeptide hormones that stimulate the proliferation and differentiation of hemopoietic precursor cells. Four murine CSFs have been identified: multi-CSF (also referred to as "IL-3"); G-CSF, M-CSF and GM-CSF. Each CSF induces the formation of a different lineage of hemopoietic cells and binds to unique receptors on progenitor cells. Similarly, four human CSFs have been identified.

Murine GM-CSF is produced by various cell types, including T-lymphocytes, macrophages, endothelial cells and fibroblasts. GM-CSF induces formation of granulocyte and macrophage colonies and activates mature eosinophils, neutrophils and macrophages. This factor has been purified from mouse lung tissue and has been shown to be a glycoprotein having a molecular weight of about 23 kilodaltons. The gene coding for murine GM-CSF now has been cloned and the entire amino acid sequence has been deduced from the observed nucleotide sequence. See, e.g., N. M. Gough, et al., (1985) The EMBO Journal, 4(3):645. The mature murine protein consists of 124 amino acids with a calculated molecular weight of about 14,000. In addition, several researchers have performed various deletion analyses to determine those regions of the protein which are important for biological activity. In particular, A. B. Shanafelt et al., (1989) Proc. Natl. Acad. Sci. (U.S.A.) 82:4360, indicates that amino acid residues 18-22, 34-41, 52-61, and 94-115 are critical to the biological activity of the murine GM-CSF. Further, N. M. Gough, et al., (1987) Eur. J. Biochem. 169:353, indicates that amino acid residues 11-15, 24-37, 47-49 and 81-89 are required for a functional murine GM-CSF molecule.

The vast majority of primary human myeloid leukemia cells obtained from blood or bone marrow sources require colony stimulating factors for proliferation and survival in vitro. For example, J. D. Griffen, et al. (1986) Blood 67:1448, found that blast cells from 13 of 15 acute myelogenous leukemic ("AML") patients grew, as colonies, in semi-solid agar when stimulated with GM-CSF. Further, several investigators have found GM-CSF dependent tumor cells in tissue samples obtained from AML patients (See, e.g., discussion in F. R. Balkwill, "Cytokines in Cancer Therapy", Oxford University Press (1989), pp. 114-149). Consequently, the ability to inhibit the growth of GM-CSF dependent tumors would be most desirable.

The present invention provides oligopeptides which are capable of inhibiting the biological activity of a GM-CSF. In particular, these oligopeptides are useful in inhibiting the growth of a GM-CSF-dependent neoplastic disease. These peptides also are useful for inhibiting the biological activity of GM-CSF on hemopoietic progenitor cells and may be useful in diseases, such as inflammatory processes, in which inhibition of this activity would be desirable.

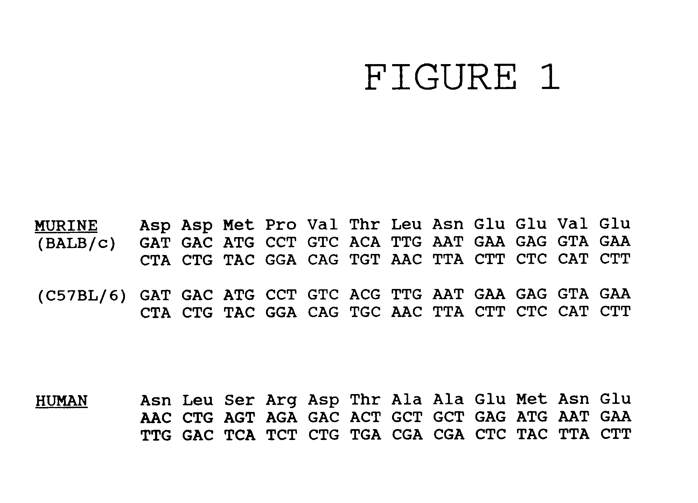

Figure 1 provides a comparison of the murine and corresponding human GM-CSF inhibiting oligopeptides of the invention. Also shown are the wild-type nucleotide sequences encoding these peptides. The oligopeptide sequences (SEQ ID NO:1 and SEQ ID NO:2, respectively) are derivable from corresponding regions of a murine and human GM-CSF, or, by using recombinant DNA methods, from the corresponding DNA coding regions. The murine region has been designated "P44-55", herein.

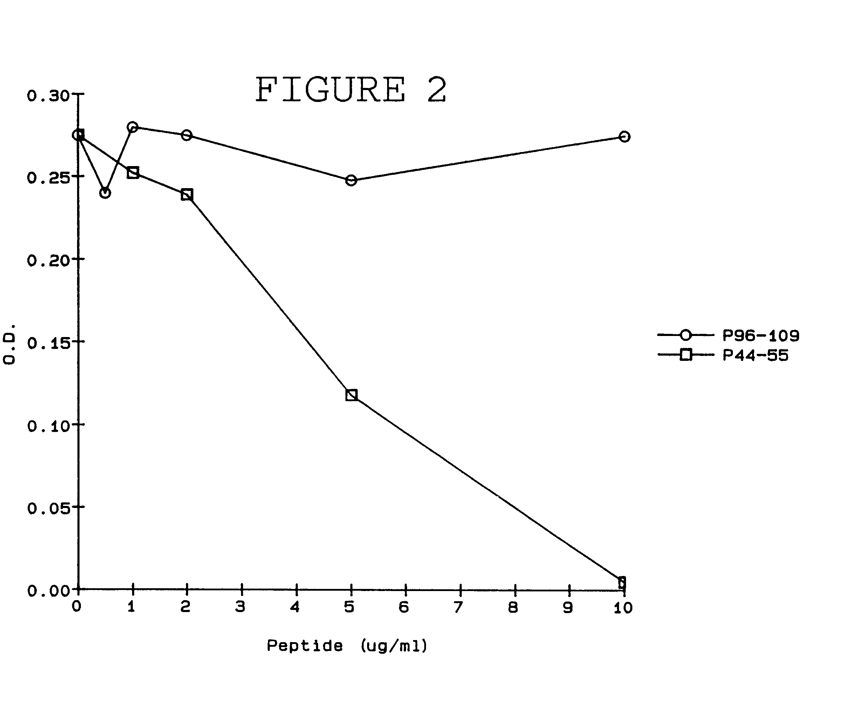

Figure 2 shows the dose-dependent inhibitory effect of an oligopeptide of the invention.

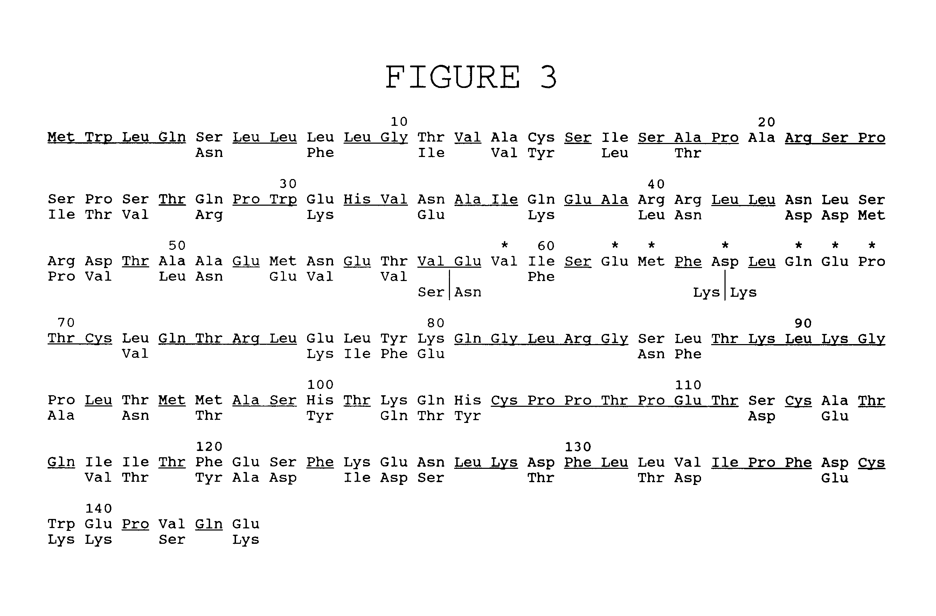

Figure 3 provides a comparison of the amino acid sequences for a human and a murine GM-CSF. Underlined amino acids are identical in both human and murine forms. Those sequences which are different in the murine form are shown below the human sequence (top line). These sequences were aligned to maximize the homology between the two forms. The vertical lines appearing at positions 57-58 and 65 of the human form are meant to indicate that that sequence does not have amino acids corresponding to the noted amino acids shown below in the murine sequence. Further, those amino acids marked with asterisks are present only in the human sequence.

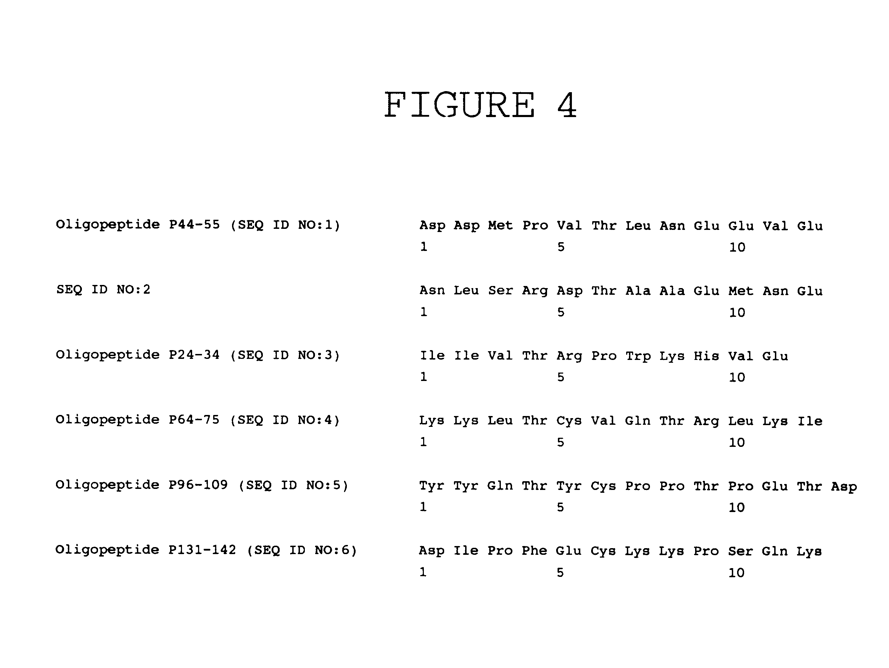

Figure 4 provides exemplary murine derivable oligopeptides useful in the present invention. The sequence denoted as SEQ ID NO:2 represents the human sequence which "corresponds" to the murine sequence designated as P44-55.

As noted, the present invention provides oligopeptides which are useful in inhibiting a biological effect of granulocyte macrophage colony stimulating factor ("GM-CSF"). In particular, an oligopeptide, the amino acid sequence of which is derivable from a mammalian GM-CSF sequence, is useful in inhibiting or preventing an undesired biological effect of GM-CSF. More specifically, an oligopeptide from a given mammalian host and which corresponds to the murine derivable sequence provided in Figure 1 (SEQ ID NO:1), is useful for inhibiting a biological effect of GM-CSF when that oligopeptide is administered to a homologous host. A preferred oligopeptide for this purpose is one which is derivable from a human GM-CSF sequence.

As used throughout, "mammalian" or "mammal" refers to any warm-blooded animal. Thus, the oligopeptide of the invention can be derivable from, for example, a murine, equine, ovine, bovine, porcine, canine, feline, or primate (including human) source. Of interest are those oligopeptides derivable from the GM-CSF sequence of laboratory animals, such as mice, rats, guinea pigs and rabbits. Further, those sequences derivable from a GM-CSF of a domestic commercial animal such as cattle, particularly, cows, sheep and pigs, will be especially useful in the present invention. A GM-CSF inhibiting oligopeptide derivable from human GM-CSF is an especially preferred embodiment of the invention. In particular, the human GM-CSF derivable oligopeptide (SEQ ID NO:2) is the most preferred embodiment of the invention.

The GM-CSF inhibiting oligopeptides of the invention are meant to be administered to a member of a "homologous" species. That is, an inhibitory peptide of the invention is to be administered to a member of the same mammalian species as that of the corresponding GM-CSF from which it is derivable. Thus, an oligopeptide derivable from a human GM-CSF sequence is to be administered to a human. Likewise, an inhibitory oligopeptide derivable from a murine GM-CSF sequence is to be administered to a murine host animal.

As used, "oligopeptide" refers to a single chain peptide, preferably containing from about 4 to about 50 amino acid residues and, most preferably, from about 4 to about 15 amino acid residues. In a preferred embodiment, the oligopeptide is one derivable from the desired GM-CSF host and corresponds to the murine inhibitory sequence provided in Figure 1 (SEQ ID NO:1).

The murine inhibitory sequence provided in Figure 1 (SEQ ID NO:1) corresponds to amino acids 21-32 of the sequence defined in Gough et al., Nature, (1984) 309:763. The predicted amino acid sequence provided in that reference resulted from analysis of cloned cDNA prepared from C57BL/6 mice and varies from that later provided in Gough et al., The EMBO Journal, (1985) 4(3):645. The murine derivable sequence of Figure 1 (SEQ ID NO:1) corresponds to amino acid residues 56-67 of this latter reference. One skilled in the art will recognize that the sequence provided in the latter reference was predicted from a full length cDNA clone prepared from BALB/c mice and probably represents an allelic variation of that provided earlier.

Thus, one skilled in the art will appreciate that variations or mutations in an oligopeptide of the present invention, as compared to a sequence found in the natural GM-CSF for a given species or subspecies, are meant to be encompassed within the scope of the present invention. As discussed in more detail below, such variations or mutations, especially when the desired oligopeptide is prepared by recombinant DNA technology, can be the result of natural, spontaneous, random or induced variation or mutation. Similarly, such variant or mutant oligopeptides can be chemically synthesized as long as the desired variant is known at the time of synthesis. In addition, smaller or larger sequences than those specifically shown in Figure 1 may possess the desired GM-CSF inhibiting activity.

Thus, as used to compare the provided murine sequence to other desired inhibitory sequences, the terms "corresponds" and "corresponding to" are meant to be flexible. In the preferred embodiments, the desired oligopeptide region of the particular host GM-CSF, when aligned for maximum homology to the murine derived GM-CSF sequence shown in Figure 1 (SEQ ID NO:1), is said to be "corresponding". Thus, Figure 1, for example, shows the human region which corresponds to the murine derivable sequence designated "P44-55", when the two full GM-CSF sequences are aligned for maximum homology as in Figure 3. As long as the oligopeptide provides the desired GM-CSF inhibitory effect, whatever the sequence, that peptide is meant to be encompassed by the present invention.

As used, terms such as "inhibitory effect", "inhibition", "capable of inhibiting", and the like, refer to an oligopeptide which decreases the observed biological effect of naturally occurring GM-CSF, from whatever source, by about 5% to about 100%. Preferably, for therapeutic administration, the decrease in observed biological activity of the GM-CSF will range from about 50% to about 100%.

The oligopeptides of the invention can be synthesized by means familiar to one skilled in the art. In particular, the amino acid sequences can be prepared synthetically, for example, by solid phase synthesis in a manner known in the art. See, e.g., Merrifield, R. B., (1963) J. Am. Chem. Soc., 85:2149; Clark-Lewis, I., et al., (1986) Science, 231:134; Kent, S., et al., (1985), in "Synthetic Peptides in Biology and Medicine" (K. Alitalo ed.), Elsevier Press, Amsterdam, p. 29; Clark-Lewis, I et al., (1987), in "Receptor Biochemistry and Methodology", (A. R. Kerlavage ed.), Alan R. Liss, New York, New York; and Clark-Lewis, I., et al., (1988), J. Immunology, 141:881. The relatively small size of the oligopeptides of the invention makes especially desirable the use of solid phase synthesis.

Generally, in solid phase peptide syntheses, the reaction occurs on an insoluble solid support, for example, a polystyrene resin to which the amino-blocked ("protected") C-terminal amino acid is attached, usually through a reactive linker moiety. Such a resin may be, for example, a tert-butyloxycarbonylaminoacyl-4-(oxymethyl)-phenylacetamido-methyl polystyrene resin ("Pam"; available from Applied Biosystems, Foster City, California). See, A. R. Mitchell, et al. (1978) J. Biol. Chem. 43:2845. The reactive group on the resin then is reacted with the carboxyl group of the C-terminal amino acid, the amino group of which has been protected. Thus, the C-terminal amino acid is covalently linked to the solid support. Upon N-deblocking of the C-terminal amino acid, the next desired N-blocked amino acid or

3

oligopeptide, or an activated form thereof, is coupled to the now free amino group of the anchored C-terminus amino acid or peptide.

Coupling can be performed with known coupling reagents, for example, N,N-diethylcarbodiimide, N,N-dicyclohexylcarbodiimide ("DCC"), carbonyldiimidazole, N-ethyl-5'-phenylisoxazolinium-3'-sulfonate or N-ethoxycarbonyl-2-ethoxy-1,2-dihydroquinoline ("EEDQ"). An activated form of the amino acid or oligopeptide to be attached means a derivative which renders the carboxyl function of the amino acid or oligopeptide reactive to coupling and formation of an amide bond with the deblocked amino group of the previously attached residue(s). Suitable activated derivatives, the means for preparing them and their use will be recognized by one skilled in the art. Preferred activated derivatives, however, may be an active ester, an anhydride, or acyl halide of the carboxy group of the desired amino acid or oligopeptide.

After the first coupling reaction, the next desired N-blocked amino acid or oligopeptide, or activated derivative thereof, is coupled to the prior existing N-deblocked amino acid or peptide which is coupled to the insoluble resin. This process is continued until the entire desired oligopeptide is synthesized. Once all of the blocking groups have been removed from the resin-anchored peptide, the peptide is cleaved from the resin by means which do not disrupt the peptidyl bonds or existing residues of the product.

Alternatively, the cleavage step may result in removal of the protecting groups present on the product. Thus, deblocking and cleavage can occur as a single step. Further, it may be possible to cleave selectively the product from the resin followed by selective deblocking of the oligopeptide of the invention.

Solid phase synthesis is especially useful for preparing the oligopeptides of the invention because these methods usually result in high yields of final product. Further, purification of the intermediate products is facilitated because the intermediate is covalently attached to the insoluble resin. Simple filtration of the resin and linked product followed by washing away of unreacted intermediates and reagents, therefore, eliminates the need to purify the desired intermediate at each step.

The solid support used in the method described may be one which is chemically inert under the condition used. Suitable supports may include, for example, resins such as styrene-divinyl benzene copolymers or polystyrenes. The resins then are modified to include reactive groups which are able to couple with the first N-protected C-terminal amino acid of the peptide to be synthesized. Such activated supports may include, for example, those which possess reactive groups such as chloromethyl groups, the "Pam" resin described earlier, 4-methyl-benzhydrylamine polystyrene, or a resin which contains reactive aminomethyl groups. Other possible "activated" resins will be apparent to one skilled in the art.

Suitable amino-protecting ("blocking") groups for use in solid phase synthesis are well known. See, for example, Protective Groups in Organic Chemistry, McOmie, ed., Plenum Press, N.Y, N.Y. (1973) and Protective Groups in Organic Synthesis, Greene, ed., John Wiley & Sons, New York, New York (1981). Such groups may include, for example, the "t-BOC" (t-butyloxycarbonyl), acetyl, benzoyl, benzyl, 2,4-dimethoxybenzyl, 4,4'-dimethoxy-benzyhdryl, phthalyl, trichloroacetyl, trifluoroacetyl, tosyl and trityl protecting groups, among others. To be suitable in the procedures outlined above, the protecting group used should remain attached to the moiety to be protected until the appropriate selective deblocking conditions are used. When deblocking is desired, then only the desired amino-protecting group(s) should be removed. Further, the deprotection should occur under reaction conditions that avoid disruption of the peptide bonds already formed.

Also, as one skilled in the art will appreciate, exposed hydroxyl, carboxy, thiol or other side chain functional groups may need to be protected during the synthesis of the peptides of the invention. Easily cleavable protecting groups for these moieties are well known and need no further elaboration. The reader is referred to "Synthetic Peptides," Volume 5, George R. Pettit, ed., Elsevier Scientific Publishing Co., Amsterdam (1980), and the references cited therein. Additionally, suitable carboxy, hydroxy, thiol and amino protecting groups are provided in references such as Protective Groups in Organic Chemistry, McOmie, ed., Plenum Press, N.Y, N.Y. (1973) and Protective Groups in Organic Synthesis, Greene, ed., John Wiley & Sons, New York, New York (1981). As is the case for the amino protecting groups, these side chain protecting groups should remain in place on the moiety to be protected until selective deblocking conditions are used to remove the blocking group. Further, the conditions used to remove the groups should be such that there is no disruption of the peptide bonds formed or so that there is no modification of existing amino acid residues of the product.

The cleavage of the final product from the resin (and any possible removal of protecting groups) generally occurs under hydrolytic conditions. Reagents used for cleavage from the resin may include, for example, hydrogen fluoride, trifluoroacetic acid, hydrogen chloride, or hydrogen bromide and trifluoroacetic acid. Alternatively, cleavage can be effected under basic conditions, by hydrazinolysis, or possibly by hydrogenolysis.

The final oligopeptide product can be purified, if desired, by standard purification methods. Such means for purification, may include, for example, gel filtration, HPLC, ion exchange chromatography, dialysis or other means known to those skilled in the art.

One skilled in the art will appreciate that the discussion above is not meant to be an exhaustive elaboration of all techniques and steps used in the chemical synthesis of peptides. Consequently, the reader is referred to the many references, such as those noted, which describe the topic in more detail. Thus, one skilled in the art may know of modifications of the methods described above that will serve equally well to prepare the inhibitory peptides of the invention. These alternate methods, likewise, are meant to be encompassed by the scope of the present invention.

In view of the discussion above, there is provided, in a further embodiment of the invention, a process for preparing a GM-CSF inhibiting oligopeptide of the invention which comprises deblocking a protected GM-CSF inhibiting oligopeptide and, if desired, purifying the product obtained.

Alternatively, the oligopeptides of the invention could be produced by recombinant DNA technology, by transformation of the desired host cell with a recombinant DNA expression vector encoding the oligopeptide of the invention, followed by culturing the host cell, under conditions suitable for expression of the desired oligopeptide, and, if desired, isolating the expressed product. The expression vector, of course, should be constructed so that the DNA sequence encoding the GM-CSF inhibiting oligopeptide, or the fusion peptide described below, is properly positioned for expression and is in proper translational reading frame in relation to the translation start site. Further, the transcription or translational start sites can be derived from the inserted DNA sequence or can be derived from other sources. These start sequences can be either heterologous or homologous to the vector, itself, if naturally-occurring, or homologous or heterologous to the promoter used in the construction. For selection purposes, the expression vector can comprise a selectable marker and transformation can be in a host cell normally sensitive to the toxic substance used for selection should the marker gene be absent.

A vector comprising a DNA sequence encoding an a GM-CSF inhibiting oligopeptide of the present invention can be expressed in a prokaryotic or eukaryotic host cell if inserted in properly designed expression vectors. For example, E. coli, Bacillus and Streptomyces expression vectors are now well-known in the art. In this regard, the desired GM-CSF inhibiting oligopeptide encoding sequence can be isolated, using commonly used recombinant DNA techniques, directly from the cDNA encoding the GM-CSF of the desired species. Such techniques may include, for example, standard restriction enzyme cleavage, gel electophoresis purification and conventional ligation techniques. In so doing, one skilled in the art will recognize that appropriate linker sequences may be used so that the desired cleavage site is placed at the appropriate location in the cDNA to be cleaved. Such linkers, depending on the sequence in question, can be ligated to a restriction fragment containing the desired GM-CSF inhibiting oligopeptide encoding gene. Alternatively, site-directed mutagenesis could be used to insert the desired restriction enzyme linker at the appropriate location of the cDNA fragment containing the GM-CSF inhibiting oligopeptide encoding gene. After such insertion, the sequence then is cleaved with the appropriate restriction enzyme.

Because the oligopeptide of the invention is of rather small size, one skilled in the art could synthesize the desired GM-CSF inhibiting oligopeptide encoding gene using standard techniques. For example, the desired single-stranded deoxyoligonucleotide sequence can be synthesized with commercially available instruments, such as a 380A DNA Synthesizer (Applied Biosystems (850 Lincoln Centre Drive, Foster City, California 94404) which uses a standard phosphoramidite chemistry. Alternatively, the desired DNA encoding sequence can be prepared by the conventional modified phosphotriester method for synthesizing single stranded DNA as described in Itakura et al., (1977) Science, 198:1056 and in Crea, et al., (1978) Proc. Natl. Acad. Sci. (U.S.A.) 75:5765. Additionally, methods for synthesizing DNA are disclosed in Hsiung et al., (1983) Nucleic Acid Research 11:3227 and Narang et al., (1980), Methods in Enzymology 68:90.

Once the desired GM-CSF inhibiting oligopeptide encoding sequence is prepared in the manner described above, it can be inserted in proper orientation for transcription and expression from a given promoter in a desired prokaryotic or eukaryotic expression vector. The expression vector then may be cloned and transformed into the desired host cell under conditions which allow selection of properly transformed host cells and then expressed, under conditions suitable for expression, of the inserted sequence, followed by recovery of the product.

Expression in a prokaryotic host cell, especially in E. coli, is not limited to the use of a particular promoter because a specific promoter is not crucial to the operability of the invention. Promoters which can be used may include, for example, the lipoprotein ("lpp") promoter, the E. coli tryptophan ("trp") promoter, bacteriophage lambda promoters, or the E. coli lactose ("lac") promoter. In addition, one or more promoters can be used in tandem, such as for example, the trp and lac promoters. In addition, hybrid promoter/translational activating sequences can be prepared, such as the tac promoter, to drive expression of the GM-CSF inhibiting oligopeptide encoding gene. All of these promoters have been previously characterized, are well-known in the art, and can be constructed either synthetically or from known plasmids.

In addition, it may be advantageous to use a thermoinducible runaway replicon such as that disclosed in U. K. Patent Publication 1,557,774 and in Uhlin et al., (1979) Gene, 6:91. At temperatures below 30°C, especially 25°C, the replicon maintains a relatively low copy number of about 10 to about 15 copies per cell. When the temperature is raised to 37°C, copy number control is lost and plasmids containing the replicon amplify to approximately 1000-2000 copies per cell.

As discussed below, the cloning of a foreign gene, such as a GM-CSF inhibiting oligopeptide encoding gene, into a vector comprising a runaway replicon may result, upon induction and loss of copy number control, in a greatly increased rate of protein synthesis and the concomitant formation of intracellular proteinaceous granules ("inclusion bodies"). The granules are highly homogeneous in their protein composition, with the desired protein product comprising up to and often exceeding 80% by dry weight of the granule. These granules are isolated easily from cell lysates and generally are stable to washing in low concentrations of urea or detergents. Washing removes proteins that bind non-specifically to the granule.

The present invention, however, does not require the use of a runaway replicon-containing plasmid for cloning and expression in a prokaryotic cell. Many replicons such as those from pBR322, pBR328, pACYC184, and the like are known in the art and are suitable for construction of recombinant DNA vectors designed to drive expression of the desired oligopeptide encoding gene.

One skilled in the art would also be familiar with the veg promoter from B. subtilis should expression in Bacillus be desired. In addition, the reader is referred to European Patent Publication, EP A 0 116 411 (published August 22, 1984), as well as U.S. Patent No. 4,559,300, both herein incorporated by reference, for their teaching of vectors and methods for expressing polypeptides in Bacillus.

In addition, U. S. Patent No. 4,559,300 discloses expression vectors for use in Streptomyces. Other such expression vectors are now well known in the art. See, for example, Horinouchi, et al., Mol. Gen. Genet., 210, 468 (1987) and Bibb, M. et al., Experimental Manipulation of Gene Expression, Ch. 4, "Developments in Streptomyces Cloning", Academic Press (1983).

In addition, the use of a particular selectable marker is not crucial to the operation of the invention. A wide variety of selectable markers exist for use in either or both of eukaryotic or prokaryotic host cells.

One skilled in the art will recognize that when expressed in a host cell, particularly a prokaryotic cell, such as, for example, E. coli, Bacillus, or Streptomyces, the small oligopeptide of the invention may undergo in vivo restriction and/or elimination from the cell. Although restrictionless host cells may be used, one skilled in the art may find it more advantageous to prepare the desired oligopeptide encoding gene so that it is linked to a cleavable linker encoding sequence which is in turn linked to a larger expressible gene so that the desired oligopeptide is expressed as a fusion product rather than as the direct expression product. For example, the desired GM-CSF inhibiting oligopeptide encoding gene may be linked at its 5' end to a DNA sequence encoding a site-specific recognition site for a protease, for example, trypsin, chymotrypsin, thermolysin, cyanogen bromide, cathepsin C, etc. which, in turn, is linked at its 5' end to a larger coding sequence, for example, a $\beta$-galactosidase, insulin, or growth hormone encoding sequence, or fragment thereof. The product expressed will be a fusion product of the structure:

Protein-{cleavage site}-GM-CSF inhibiting oligopeptide

One skilled in the art will appreciate that the desired specific cleavage site should not also appear in the oligopeptide region of the fusion product. After isolation of the noted fusion product, the polypeptide is treated with the appropriate protease which recognizes the inserted specific protease cleavage site. This will liberate the GM-CSF inhibiting oligopeptide of the invention which then can be isolated by conventional means. Other means to avoid in vivo degradation of an expressed oligopeptide of the invention may be apparent to one skilled in the art and use of these other methods to accomplish the same result are meant to be encompassed by the present invention.

In a prokaryote, especially E. coli, the product, especially a fusion product of the type described above, may be deposited within the host cell as "inclusion bodies". The purification of the product, in such a case, may require the disruption of the host cell membrane, denaturation of the product, and, if desired, as noted above, the cleavage of any desired portion of the product by use of an appropriate protease. The purification, denaturation and cleavage of the inclusion bodies is well known and need not be elaborated further.

The preferred host cell for cloning and expression, using recombinant DNA means, is a prokaryotic cell, preferably E. coli. One skilled in the art, however, may want to express in a eukaryotic host cell the GM-CSF inhibiting oligopeptide encoding gene. Suitable eukaryotic host cells may be, for example, a mammalian cell or perhaps a yeast cell such as Saccharomyces, Kluyveromyces or Pichia. Eukaryotic host cells useful for this purpose may include, for example, the following: Human 293 (ATCC CRL 1573); Syrian Hamster AV12-664 (ATCC CRL 9595); Chinese Hamster Ovary (CHO-K1--ATCC CCL 61); 3T3 (Mouse Embryo Fibroblast--ATCC CCL 92); CV-1 (African Green Monkey Kidney--ATCC CCL 70); LLC-MK$_2$ - (Rhesus Monkey Kidney--ATCC CCL 7); HepG-2 (Human Liver Hepatoblastoma--ATCC HB 8065); COS-1 (Monkey Kidney--SV40 Transformed--ATCC CRL 1650); BHK-21 (Syrian Hamster Kidney--ATCC CCL 10), as well as other eukaryotic cell lines which are publically available from sources such as the American Type Culture Collection ("ATCC"), Rockville, MD. A wide variety of vectors exist for the transformation of such eukaryotic host cells and the description below is in no way meant to be limiting on the means for expression of or products encompassed within the scope of the invention.

For example, a wide variety of pSV2-type vectors comprise segments of the SV40 genome and which constitute a defined eukaryotic transcription unit including a promoter (e.g. "EP", early promoter), intervening sequences ("IVS"), and polyadenylation ("PA") sites. In the absence of the SV40 T-antigen, the pSV2-type vectors transform mammalian and other eukaryotic host cells by integrating into the host cell chromosomal DNA. A variety of plasmid pSV2-type vectors have been constructed (See, Eukaryotic Viral Vectors, edited by Gluzman, Cold Spring Harbor Laboratories, N.Y. 1982) such as plasmids pSV2-gpt, pSV2-neo, pSV2-dhfr, and pSV2-$\beta$-globin, in which the SV40 promoter drives transcription of an inserted gene. The construction and use of these vectors is now well known to those skilled in the art. Such vectors are available from the American Type Culture Collection ("ATCC"), Rockville, MD or from the Northern Regional Research Laboratory ("NRRL"), Peoria, Illinois.

Furthermore, other plasmids useful for expressing the oligopeptides of the invention can use promoters other than the SV40 early promoter. The present invention is in no way limited to the use of any particular promoter. Other promoters, such as the SV40 late promoter or promoters from other eukaryotic genes, such as, for example, the estrogen-inducible chicken ovalbumin gene, the interferon genes, the glucocorticoid-inducible tyrosine aminotransferase gene, the thymidine kinase gene and the major early and late adenovirus genes, can be readily isolated and modified for use on recombinant DNA expression vectors designed to produce an oligopeptide of the invention. Eukaryotic promoters can be used in tandem to drive expression of the desired final product. All that is necessary is that the particular promoter, associated with the inserted gene sequence, function in the host cell into which the vector is transformed.

Additionally, a large number of retroviruses are known which infect a wide range of eukaryotic host cells. Long terminal repeats ("LTR") in retroviral DNA often encode promoter activity and can be used in place of the SV40 early promoter, described above, to drive transcription and translation of the oligopeptide encoding gene. For example, plasmid pRSVcat (available from the ATCC under the accession number ATCC 37152) comprises portions of the LTR of Rous Sarcoma Virus ("RSV"), a virus known to infect chicken and other host cells. The RSV LTR sequences can be isolated on an ~.76 kb NdeI-HindIII restriction fragment of plasmid pRSVcat. This promoter can be isolated and inserted in an appropriate vector such that it is positioned correctly to drive transcription and expression of the desired oligopeptide encoding sequence.

Further, other eukaryotic or mammalian expression systems are known. For example, the reader is referred to published European Patent Application No. 87303083.7, published as EP A 0 245 949 (published November 19, 1987) and corresponding to U. S. Ser. No. 06/849999 (filed April 9, 1986), incorporated herein by reference. A particularly useful eukaryotic expression vector is plasmid phd. Plasmid phd is a convenient eukaryotic expression vector because it is a cassette vector which contains the BK virus enhancer sequence ("BK") immediately upstream from the adenovirus-2 late promoter ("AV2LP"). In addition, plasmid phd contains a single BclI restriction enzyme recognition sequence positioned for insertion of a desired DNA sequence so that it is expressed from the BK enhancer-AV2LP promoter system. This construction allows for high level transcription and subsequent expression of an inserted gene sequence. Plasmid phd also contains a hygromycin resistance-conferring sequence, as well as a dihydrofolate reductase ("dhfr") cistron, both of which can be used as selectable markers in hygromycin sensitive or dhfr negative host cells, respectively. The construction of plasmid phd is described in detail in European Patent Application, EP A 0 245 949 (Appln. No. 87303083.7), published November 19, 1987. Plasmid phd has been transformed into E. coli K12 GM48, the preferred source and stock reservoir of the plasmid. The plasmid can be isolated by conventional means from this strain which has been deposited and made a part of the NRRL permanent stock culture collection. The strain is available under the accession number NRRL B-18525.

Thus, one may isolate plasmid phd and cleave it with BclI restriction enzyme. Similarly, an oligopeptide encoding sequence, or preferably a fusion peptide encoding region, including the necessary translation start site, such as that described earlier, is prepared, BclI linkers attached, and the fragment obtained is cleaved with BclI restriction endonuclease. The phd fragment and the oligopeptide encoding fragment are ligated to obtain a useful expression vector capable of expressing the desired oligopeptide, or oligopeptide fusion product. Upon transformation and culturing of the desired eukaryotic host cell, under conditions suitable for expression of the inserted gene sequence, one skilled in the art will be able to obtain the desired oligopeptide, either as a fusion protein, if additional DNA encoding sequences are added as described above, or as the direct expression product. Isolation, purification and cleavage, if necessary, of the expressed product have been described above or will be apparent to one skilled in the art.

Also, should expression in a yeast cell be desirable, the means for preparing yeast expression systems are now well described in the art. The reader is referred to the following references if such expression is desired: U. S. Patent No. 4,775,622 (issued October 4, 1988); European Patent No. EP B 0 073 673 (granted April 20, 1988); U. S. Patent No. 4,615,974 (issued October 7, 1986); and European Patent Publication EP A 0 183 070 (published June 4, 1986).

As noted, the eukaryotic expression vectors described or any other vector constructed can be transformed into and expressed in a variety of eukaryotic, especially mammalian, host cells. Vectors which contain no selectable marker with which to isolate and identify stable transformants, would be useful for transient assay or for purposes of cotransformation, such as in the procedure outlined in U. S. Patent No. 4,399,216, issued August 26, 1983, incorporated herein by reference. The vectors used may be expression "shuttle" vectors and may include sequences which allow for efficient replication in E. coli. Such shuttle vectors are useful because it is usually more efficient to prepare plasmid DNA in E. coli than in other host organisms. Then, if desired, the vectors are transformed into the ultimately desired host.

Should one skilled in the art decide to use recombinant technology to produce the oligopeptides of the invention, it will be clear that the specific vectors described above are not to be considered as the only way to clone and express the DNA sequences encoding the GM-CSF inhibiting oligopeptides of the invention. In particular, one skilled in the art now can construct vectors containing any desired restriction enzyme recognition sequence(s) to prepare both eukaryotic and prokaryotic recombinant DNA vectors which are useful for preparing the desired oligonucleotide. In addition, one skilled in the art is fully familiar with the degeneracy of the genetic code. Consequently, the skilled artisan can modify the DNA sequences used in the present invention to provide homologous proteins having the same or improved physiological characteristics compared to the specific oligopeptides provided in Figure 1. Also, one skilled in the art can modify the DNA sequences to express an identical protein to those provided by the invention, albeit expressed at higher levels.

Furthermore, one skilled in the art is familiar with means to prepare synthetically, either partially, or in whole, DNA sequences which would be useful in preparing recombinant DNA vectors or oligopeptide encoding sequences to prepare products which are within the scope of the present invention. Additionally, recombinant means for modifying the DNA sequences provided may include, for example, site-directed mutagenesis. These techniques are well known to those skilled in the art and require no further elaboration here. Consequently, any "constructed" DNA or vector includes within its scope DNA which is isolated from natural sources, prepared synthetically or semi-synthetically (from natural and synthetic sources), or which are modified by recombinant DNA methods.

Likewise, as noted earlier, the oligopeptides can be prepared, in whole or in part, by conventional solid phase synthesis. The final desired oligopeptide could be prepared using a combination of recombinant and non-recombinant techniques. Thus, the present invention should not be construed as limited to the specific means for producing the desired GM-CSF inhibiting oligopeptides of the invention.

Further, as noted, the sequences used to arrive at the oligopeptides provided in Figure 1 are those derived directly from the full length natural GM-CSF protein. One skilled in the art, however, can modify these sequences, or the DNA encoding such sequences, to prepare other inhibitory oligopeptides of the invention. Consequently, "derivable", as used herein, is meant to encompass these modified products. "Derivable" also is meant to indicate that the original source of the particular amino acid sequence for the final oligopeptide is the GM-CSF polypeptide, a portion thereof, or a corresponding DNA coding region. This is so even though, through deliberate design, one skilled in the art makes modifications, deletions, or additions to the original natural sequence in question.

As described in more detail in the examples, the GM-CSF inhibiting oligopeptides of the invention are useful in the treatment of disease states which are mediated by (i.e. which involve at some stage in a biochemical pathway or which require) GM-CSF for continued growth and progression. In particular, one skilled in the art will appreciate that it is possible to inhibit or prevent undesirable disease states which exist by virtue of the GM-CSF colony stimulating activity on hemopoietic progenitor cells, for example, bone marrow precursor cells, such as macrophage or granulocyte precursors. Such conditions may include, for example, disease states which involve excess production of macrophages and/or granulocytes such as inflammatory diseases and various auto-immune diseases. Specific disease states which involve such inflammatory processes may include, for example, temporal arteritis, poly-arteritis nodosa, systemic lupus erythematosis, polymyalgia rheumatica, various forms of nephritis and possibly atherosclerosis.

Further, as elaborated earlier, the oligopeptides of the invention could be useful for inhibiting or eliminating the growth of a GM-CSF dependent neoplastic disease. In a preferred aspect of the invention, a GM-CSF inhibiting oligopeptide can inhibit or eliminate the growth of a GM-CSF dependent lymphoma or leukemia.

Thus, the present invention provides in another embodiment a method for alleviating in a warm-blooded mammal in need thereof an undesirable effect of GM-CSF, which comprises administering to said mammal a GM-CSF inhibiting amount of an oligopeptide of the invention.

In an additional embodiment, there is provided a method for inhibiting or eliminating the growth of a GM-CSF dependent neoplastic disease in a warm-blooded mammal which comprises administering to said mammal a GM-CSF inhibiting oligopeptide of the invention.

There are also provided formulations useful for the administration of the oligopeptides of the invention. In particular, the present invention provides a pharmaceutical formulation which comprises an oligopeptide which is capable of inhibiting the biological activity of GM-CSF associated with a pharmaceutically acceptable carrier, diluent or excipient therefor. Preferably, the oligopeptides of the invention are prepared as formulations suitable for parenteral administration, for example, injection or I.V. administration, or by other methods so as to ensure their delivery to the bloodstream in an effective form. Such formulations preferably are in unit dosage form, each dosage containing, for example, from about 0.001 to about 1000 mg of the desired oligopeptide. Preferably, the formulations will contain from about .01 to about 200 mg of the oligopeptide.

The means for formulating oligopeptides of the present invention will be well known to one skilled in the art. In particular, an oligopeptide of the invention may be combined in admixture with a pharmaceutically acceptable carrier, diluent or carrier. Suitable carriers, diluents or excipients and their formulation, inclusive of other human proteins, e.g. human serum albumin, are described, for example, in Remington's Pharmaceutical Sciences, 16th ed., 1980, Mack Publishing Co., edited by Osol et al., which is incorporated by reference. Typical pharmaceutically acceptable excipients, diluents and carriers, especially for parenteral administration, the preferred route of administration, may include, for example, isotonic saline, dilute aqueous dextrose (e.g. 5%), the polyhydric aliphatic alcohols or mixtures thereof, for instance glycerin, propylene glycol, polyethylene glycol, and the like. Parenteral solutions may contain also a preservative such as phenethyl alcohol, methyl or propyl paraben, thimerosal and the like. If needed, about 0.05 to about 0.20 per cent by weight of an antioxidant such as sodium metabisulfite or sodium bisulfite can be employed also. "Pharmaceutically acceptable" refers to those excipients, diluents or carriers which are useful in the treatment or diagnosis of a warm-blooded mammal.

As one skilled in the art will appreciate, the oligopeptides of the invention will be effective over a wide dosage range depending on factors such as the disease state being treated, the manner of administration, the age and condition of the patient as well as other factors commonly determined and considered by the administering physician.

The following non-limiting examples are provided to further illustrate and describe the invention. The invention is not to be construed as limited in scope by reason of the descriptions given in any of the examples. Sources of reagents are provided for convenience and are not meant to limit the invention.

Preparation 1

Synthesis of Oligopeptides

Oligopeptides P44-55 (SEQ ID NO:1), P24-34 (SEQ ID NO:3), P64-75 (SEQ ID NO:4), P96-109 (SEQ ID NO:5) and P131-142 (SEQ ID NO:6) are synthesized by solid phase synthesis as described earlier in the specification. The products, when analyzed by HPLC under the noted conditions have the following retention times:

| OLIGOPEPTIDE | OBSERVED RETENTION TIME (MINUTES) |
|---|---|
| P44-55 (SEQ ID NO:1) | 3.1* |
| P24-34 (SEQ ID NO:3) | 4.6** |
| P64-75 (SEQ ID NO:4) | 21.2** |
| P96-109 (SEQ ID NO:5) | 20.1** |
| P131-142 (SEQ ID NO:6) | 35.7** |

*$C_{18}$ Reverse phase column (4.6 cm x 25 cm, Beckman Instruments); Linear gradient 10% buffer B (90% Buffer A) to 50% buffer B (50% Buffer A) (1ml/min)[Buffer A: 10 mM ammonium acetate (pH 6.0); Buffer B: 10 mM ammonium acetate/acetonitrile (10:90)]; Detection at 220 nm.

**$C_8$ (6μ) Dupont Zorbax column; Gradient 5-95% Buffer B (Buffer A 95% to 5%) in 50 minutes [Buffer A: 0.1% TFA; Buffer B: Acetonitrile:$H_2O$ (80:20), 0.1% TFA]; Detection at 214 nm.

Example 1

GM-CSF Activity on bone marrow precursors

To determine the effect of the desired inhibitory oligopeptide, the following assay, using murine bone marrow precursors, was developed. Of course, if using an oligopeptide derivable from a species other than murine, substitution of cells from that species into the assay procedure would be required.

The biological activity of GM-CSF was determined in a liquid culture system of murine bone marrow cells (see, e.g., I. Bursuker, et al., J. Reticuloendothelial Soc., 25, 533 (1979)). Bone marrow cells were collected from femurs and tibia of 10 to 12 week old CB6F1 (C57BLxBalb/c) male mice (Harlan-Sprague Dawley, Inc., Indianapolis, Indiana) by flushing dissected and clean bones with phosphate buffered saline ("PBS", GIBCO, Grand Island, New York). Viable cell count was determined using trypan blue. The cells then were plated (1 ml/well; 2 X $10^5$ cells/ml) in 24-well tissue culture plates (Costar, Cambridge, MA). Granulocyte-macrophage colony stimulating factor ("GM-CSF"; 2 ng/ml; recombinantly produced and available from PeproTech, Inc., Rocky Hill, NJ) and the desired oligopeptide were added at the desired concentration to the wells. The bone marrow cells were cultured for 6 days in a $CO_2$ incubator at 37° C. At the end of the culture period, the plates were washed with PBS which left in the well only mature adherent granulocytes and macrophages. The cells then were fixed with 5% formaldehyde for 20 minutes at 4° C, washed with 0.1 M borate buffer (pH 8.5), and stained with 1% methylene blue in the same buffer for 10 minutes at room temperature. After a thorough washing in the borate buffer, the dye was eluted from the cells with 0.1 N HCl for 30 minutes at 42° C. The dye absorbance was measured at 620 nm in a MCC/340 Titertech Multiscan plate reader (Eflab, Helsinki, Finland). The dye absorbance reading is proportional to the number of cells in the culture.

The results of this assay, using 5 different oligopeptides spanning various regions of the murine GM-CSF molecule, are provided in Table 1.

TABLE 1

EFFECT OF PEPTIDES ON GM-CSF ACTIVITY

| PEPTIDES[a] | %INHIBITION |
|---|---|
| P24-34 (SEQ ID NO:3) | 4.8 |
| P44-55 (SEQ ID NO:1) | 98.9 |
| P64-75 (SEQ ID NO:4) | 3.6 |
| P96-109 (SEQ ID NO:5) | 0.0 |
| P131-142 (SEQ ID NO:6) | -3.6 |

[a]The desired oligopeptide was added to the culture at a concentration of 10 μg/ml

The results in Table 1 show that oligopeptide P44-55 (SEQ ID NO:1), at a concentration of 10 μg/ml, completely inhibits the biological activity of GM-CSF.

Further analysis shows that the inhibitory effect of oligopeptide P44-55 on GM-CSF-induced granulocyte macrophage formation is dose-dependent within the range of about 1 μg/ml to about 10 μg/ml. Another oligopeptide, P96-109, did not affect the biological activity of GM-CSF at any of the test concentrations. These results are shown in Figure 2.

Example 2

11

Inhibition of Proliferation of a GM-CSF Dependent Transformed Cell Line

To determine the ability of an oligopeptide of the invention to inhibit the growth and proliferation of a GM-CSF dependent transformed cell line, the following screen was developed. As noted previously, if using an oligopeptide derivable from a species other than murine, substitution of cells from that species into the screening procedure would be desirable.

A. Culture and Maintenance of a GM-CSF Dependent Transformed Cell Line

A murine GM-CSF dependent transformed cell line, HT-2 (See, J. Watson, J. Exp. Med., 150, 1510 (1979)), was obtained from Dr. Yacov Ron (University of Medicine and Dentistry of New Jersey, Piscataway, NJ). Other GM-CSF dependent transformed cell lines could be substituted. For example, for screening of oligopeptides which inhibit the human form of GM-CSF, the following GM-CSF dependent human cell lines could be substituted in the present screen: TALL-101, AML-193, MV4-11. These cell lines are described in D. Santoli et al., J. Immunol. 139:3348-3354 (1987). The HT-2 cells were grown in RPMI 1640 medium (GIBCO, Grand Island, NY) supplemented with 10% fetal bovine serum (HyClone, Logan, UT), antibiotics (penicillin (100 units/ml) and streptomycin (100 $\mu$g/ml)) and 10 ng/ml of recombinantly produced murine GM-CSF (PeproTech, Inc., Rocky Hill, NJ).

B. $^3$H-Thymidine Incorporation Assay

HT-2 cells (1-2 x $10^4$/0.1 ml) were plated into 96-well microtiter plates in RPMI 1640 medium with 10% fetal bovine serum. GM-CSF and the desired oligopeptide were added to the wells to make up a final volume of 0.2 ml. The cells then were cultured for 4 hours at 37°C in a $CO_2$ incubator and pulse-labeled for 4 hours by adding 1 $\mu$Ci/well of [3H-methyl]-thymidine in a 25 $\mu$l volume. The plates were harvested using a Tomtech Harvester 96 (Orange, CT) apparatus and the filters obtained were counted in a LKB-$\beta$ plate reader.

The results of incubating HT-2 cells in the presence of oligopeptide P44-55 (SEQ ID NO:1) are provided in Table 2.

EP 0 499 162 A2

Table 2

Inhibition of GM-CSF Induced Proliferation of HT-2 Cells by oligopeptide P44-55[a]

| Oligopeptide Concentration (µg/ml) | %Inhibition |
|---|---|
| 0 | 0 |
| 0.12 | 28 |
| 0.5 | 64 |
| 2.0 | 78 |

[a]HT-2 cells were incubated in the presence of 2 ng/ml of GM-CSF. oligopeptide P44-55 was added at the noted concentrations. P131-142 (SEQ ID NO:6) was inactive in this screen.

The results provided in Table 2 show that oligopeptide P44-55 (SEQ ID NO:1) inhibits, in a dose-dependent manner, the GM-CSF induced proliferation of the murine tumor line.

The invention has been described with particular reference to specific embodiments, but it will be understood that variations and modifications can be made which are meant to fall within the scope and spirit of the invention.

13

SEQUENCE LISTING

(1)  GENERAL INFORMATION:

     (i)  APPLICANT:  Bursuker, I
                      Greenfield, Robert S
                      Braslawsky, Gary R

     (ii)  TITLE OF INVENTION:   GM-CSF Inhibiting Oligopeptides

     (iii)  NUMBER OF SEQUENCES:  6

     (iv)  CORRESPONDENCE ADDRESS:
           (A)  ADDRESSEE:  Bristol-Myers Squibb Company
           (B)  STREET:  P.O. Box 5100
           (C)  CITY:  Wallingford
           (D)  STATE:  Connecticut
           (E)  COUNTRY:  USA
           (F)  ZIP:  06492-7660

     (v)  COMPUTER READABLE FORM:
          (A)  MEDIUM TYPE:  Diskette-3.50 inch, 1.44 Mb storage
          (B)  COMPUTER:  IBM PS/2
          (C)  OPERATING SYSTEM:  PC-DOS
          (D)  SOFTWARE: WordPerfect 5.1

     (viii)  ATTORNEY/AGENT INFORMATION:
             (A)  NAME:  Levy, Ron K
             (B)  REGISTRATION NUMBER:  31539
             (C)  REFERENCE/DOCKET NUMBER:  CT-2116

     (ix)  TELECOMMUNICATION INFORMATION:
           (A)  TELEPHONE:  203 284 6244

(2)  INFORMATION FOR SEQ ID NO:1:

     (i)  SEQUENCE CHARACTERISTICS:
          (A)  LENGTH: 12 amino acids
          (B)  TYPE: amino acid
          (D)  TOPOLOGY: linear

     (ii)  MOLECULE TYPE: peptide

     (xi)  SEQUENCE DESCRIPTION: SEQ ID NO:1:

     Asp Asp Met Pro Val Thr Leu Asn Glu Glu Val Glu
     1               5                   10

14

(2)     INFORMATION FOR SEQ ID NO:2:

        (i) SEQUENCE CHARACTERISTICS:
            (A)   LENGTH: 12 amino acids
            (B)   TYPE: amino acid
            (D)   TOPOLOGY: linear

        (ii) MOLECULE TYPE: peptide

        (xi) SEQUENCE DESCRIPTION: SEQ ID NO:2:

Asn Leu Ser Arg Asp Thr Ala Ala Glu Met Asn Glu
1               5                   10

(2)     INFORMATION FOR SEQ ID NO:3:

        (i) SEQUENCE CHARACTERISTICS:
            (A)   LENGTH: 11 amino acids
            (B)   TYPE: amino acid
            (D)   TOPOLOGY: linear

        (ii) MOLECULE TYPE: peptide

        (xi) SEQUENCE DESCRIPTION: SEQ ID NO:3:

Ile Ile Val Thr Arg Pro Trp Lys His Val Glu
1               5                   10

(2)     INFORMATION FOR SEQ ID NO:4:

        (i) SEQUENCE CHARACTERISTICS:
            (A)   LENGTH: 12 amino acids
            (B)   TYPE: amino acid
            (D)   TOPOLOGY: linear

        (ii) MOLECULE TYPE: peptide

        (xi) SEQUENCE DESCRIPTION: SEQ ID NO:4:

Lys Lys Leu Thr Cys Val Gln Thr Arg Leu Lys Ile
1               5                   10

```
(2)     INFORMATION FOR SEQ ID NO:5:

           (i) SEQUENCE CHARACTERISTICS:
                 (A)   LENGTH: 13 amino acids
                 (B)   TYPE: amino acid
                 (D)   TOPOLOGY: linear

          (ii) MOLECULE TYPE: peptide

          (xi) SEQUENCE DESCRIPTION: SEQ ID NO:5:

                Tyr Tyr Gln Thr Tyr Cys Pro Pro Thr Pro Glu Thr Asp
                1               5                   10

(2)     INFORMATION FOR SEQ ID NO:6:

           (i) SEQUENCE CHARACTERISTICS:
                 (A)   LENGTH: 12 amino acids
                 (B)   TYPE: amino acid
                 (D)   TOPOLOGY: linear

          (ii) MOLECULE TYPE: peptide

          (xi) SEQUENCE DESCRIPTION: SEQ ID NO:6:

                Asp Ile Pro Phe Glu Cys Lys Lys Pro Ser Gln Lys
                1               5                   10
```

## Claims

**Claims for the following Contracting States : AT, BE, CH, LI, DE, DK, FR, GB, IT, LU, MC, NL, PT, SE**

1. An oligopeptide which is capable of inhibiting the biological activity of a GM-CSF.

2. An oligopeptide as claimed in Claim 1 which is capable of inhibiting the effect of GM-CSF on a hemopoietic progenitor cell.

3. An oligopeptide as claimed in Claim 1 or 2 which is capable of inhibiting the effect of GM-CSF on a bone marrow precursor of granulocytes.

4. An oligopeptide as claimed in Claim 1 or 2 which is capable of inhibiting the effect of GM-CSF on a bone marrow precursor of macrophages.

5. An oligopeptide as claimed in Claim 1 which is capable of inhibiting the growth of a GM-CSF dependent neoplastic disease.

6. An oligopeptide as claimed in any one of Claims 1 to 5 which is capable of inhibiting the biological activity of human GM-CSF.

7. A GM-CSF inhibiting oligopeptide as claimed in Claim 6 which comprises the sequence (SEQ ID NO:2):

   Asn Leu Ser Arg Asp Thr Ala Ala Glu Met Asn Glu.

8. A GM-CSF inhibiting oligopeptide of Claim 7 which is (SEQ ID NO:2):

   Asn Leu Ser Arg Asp Thr Ala Ala Glu Met Asn Glu.

9. A pharmaceutical formulation which comprises as an active ingredient an oligopeptide, as claimed in any one of Claims 1 to 8, associated with a pharmaceutically acceptable carrier, diluent or excipient therefor.

10. An oligopeptide, as claimed in any one of the Claims 1 to 8, for use in therapy.

11. A process for preparing an oligopeptide as claimed in any one of Claims 1 to 8 which comprises deblocking a protected GM-CSF inhibiting oligopeptide, and, if desired, purifying the product.

**Claims for the following Contacting State : ES**

1. A process for preparing an oligopeptide which is capable of inhibiting the biological activity of a GM-CSF, which comprises deblocking by hydrolytic or hydrogenolytic means a protected GM-CSF inhibiting oligopeptide, and, if desired, purifying the product.

2. A process as claimed in Claim 1 in which the oligopeptide is capable of inhibiting the effect of GM-CSF on a hemopoietic progenitor cell.

3. A process as claimed in Claim 1 or 2 in which the oligopeptide is capable of inhibiting the effect of GM-CSF on a bone marrow precursor of granulocytes.

4. A process as claimed in Claim 1 or 2 in which the oligopeptide is capable of inhibiting the effect of GM-CSF on a bone marrow precursor of macrophages.

5. A process as claimed in Claim 1 in which the oligopeptide is capable of inhibiting the growth of a GM-CSF dependent neoplastic disease.

6. A process as claimed in any one of Claims 1 to 5 in which the oligopeptide is capable of inhibiting the biological activity of human GM-CSF.

7. A process as claimed in Claim 6 in which the GM-CSF inhibiting oligopeptide comprises the sequence (SEQ ID NO:2):

   Asn Leu Ser Arg Asp Thr Ala Ala Glu Met Asn Glu.

8. A process as claimed in Claim 7 in which the GM-CSF inhibiting oligopeptide is (SEQ ID NO:2):

   Asn Leu Ser Arg Asp Thr Ala Ala Glu Met Asn Glu.

9. A process for preparing a pharmaceutical formulation which comprises admixing an oligopeptide, as defined in any one of Claims 1 to 8, with a pharmaceutically acceptable carrier, diluent or excipient therefor.

10. The use of an oligopeptide, as defined in any one of the Claims 1 to 8, for preparing a pharmaceutical formulation for inhibiting GM-CSF.

**Claims for the following Contracting State : GR**

1. An oligopeptide which is capable of inhibiting the biological activity of a GM-CSF.

2. An oligopeptide as claimed in Claim 1 which is capable of inhibiting the effect of GM-CSF on a hemopoietic progenitor cell.

3. An oligopeptide as claimed in Claim 1 or 2 which is capable of inhibiting the effect of GM-CSF on a bone marrow precursor of granulocytes.

4. An oligopeptide as claimed in Claim 1 or 2 which is capable of inhibiting the effect of GM-CSF on a bone marrow precursor of macrophages.

5. An oligopeptide as claimed in Claim 1 which is capable of inhibiting the growth of a GM-CSF dependent neoplastic disease.

6. An oligopeptide as claimed in any one of Claims 1 to 5 which is capable of inhibiting the biological activity of human GM-CSF.

7. A GM-CSF inhibiting oligopeptide as claimed in Claim 6 which comprises the sequence (SEQ ID NO:2):

Asn Leu Ser Arg Asp Thr Ala Ala Glu Met Asn Glu.

8. A GM-CSF inhibiting oligopeptide of Claim 7 which is (SEQ ID NO:2):

Asn Leu Ser Arg Asp Thr Ala Ala Glu Met Asn Glu.

9. A process for preparing a pharmaceutical formulation which comprises admixing an oligopeptide, as claimed in any one of Claims 1 to 8, with a pharmaceutically acceptable carrier, diluent or excipient therefor.

10. An oligopeptide, as claimed in any one of the Claims 1 to 8, for use in therapy.

11. A process for preparing an oligopeptide as claimed in any one of Claims 1 to 8 which comprises deblocking a protected GM-CSF inhibiting oligopeptide, and, if desired, purifying the product.

# FIGURE 1

EP 0 499 162 A2

```
MURINE     Asp Asp Met Pro Val Thr Leu Asn Glu Glu Val Glu
(BALB/c)   GAT GAC ATG CCT GTC ACA TTG AAT GAA GAG GTA GAA
           CTA CTG TAC GGA CAG TGT AAC TTA CTT CTC CAT CTT

(C57BL/6)  GAT GAC ATG CCT GTC ACG TTG AAT GAA GAG GTA GAA
           CTA CTG TAC GGA CAG TGC AAC TTA CTT CTC CAT CTT



HUMAN      Asn Leu Ser Arg Asp Thr Ala Ala Glu Met Asn Glu
           AAC CTG AGT AGA GAC ACT GCT GCT GAG ATG AAT GAA
           TTG GAC TCA TCT CTG TGA CGA CGA CTC TAC TTA CTT
```

FIGURE 2

# FIGURE 3

```
                                       10                                  20
Met Trp Leu Gln Ser Leu Leu Leu Leu Gly Thr Val Ala Cys Ser Ile Ser Ala Pro Ala Arg Ser Pro
            Asn         Phe         Ile     Val Tyr     Leu     Thr

                             30                              40
Ser Pro Ser Thr Gln Pro Trp Glu His Val Asn Ala Ile Gln Glu Ala Arg Arg Leu Leu Asn Leu Ser
Ile Thr Val     Arg     Lys         Glu     Lys         Leu Asn         Asp Asp Met

                 50                          *   60      *   *       *       *   *   *
Arg Asp Thr Ala Ala Glu Met Asn Glu Thr Val Glu Val Ile Ser Glu Met Phe Asp Leu Gln Glu Pro
Pro Val     Leu Asn     Glu Val     Val         Phe             Lys Lys
                                    Ser|Asn

70                              80                              90
Thr Cys Leu Gln Thr Arg Leu Glu Leu Tyr Lys Gln Gly Leu Arg Gly Ser Leu Thr Lys Leu Lys Gly
        Val         Lys Ile Phe Glu             Asn Phe

                     100                          110
Pro Leu Thr Met Met Ala Ser His Thr Lys Gln His Cys Pro Pro Thr Pro Glu Thr Ser Cys Ala Thr
Ala     Asn     Thr     Tyr     Gln Thr Tyr             Asp     Glu

                 120                          130
Gln Ile Ile Thr Phe Glu Ser Phe Lys Glu Asn Leu Lys Asp Phe Leu Leu Val Ile Pro Phe Asp Cys
    Val Thr     Tyr Ala Asp     Ile Asp Ser     Thr     Thr Asp             Glu

         140
Trp Glu Pro Val Gln Glu
Lys Lys     Ser     Lys
```

# FIGURE 4

Oligopeptide P44-55 (SEQ ID NO:1)    Asp Asp Met Pro Val Thr Leu Asn Glu Glu Val Glu
    1        5        10

SEQ ID NO:2    Asn Leu Ser Arg Asp Thr Ala Ala Glu Met Asn Glu
    1        5        10

Oligopeptide P24-34 (SEQ ID NO:3)    Ile Ile Val Thr Arg Pro Trp Lys His Val Glu
    1        5        10

Oligopeptide P64-75 (SEQ ID NO:4)    Lys Lys Leu Thr Cys Val Gln Thr Arg Leu Lys Ile
    1        5        10

Oligopeptide P96-109 (SEQ ID NO:5)    Tyr Tyr Gln Thr Tyr Cys Pro Pro Thr Pro Glu Thr Asp
    1        5        10

Oligopeptide P131-142 (SEQ ID NO:6)    Asp Ile Pro Phe Glu Cys Lys Lys Pro Ser Gln Lys
    1        5        10

EP 0 499 162 A2